(19) Europäisches Patentamt
European Patent Office
Office européen des brevets

(11) Veröffentlichungsnummer: 0 224 779
A1

(12) # EUROPÄISCHE PATENTANMELDUNG

(21) Anmeldenummer: 86115879.8

(22) Anmeldetag: 15.11.86

(51) Int. Cl.[4]: C 07 D 493/06
C 07 D 471/06, C 08 K 5/15
C 08 K 5/34
//(C07D493/06, 311:00, 311:00),
(C07D471/06, 221:00, 221:00)

(30) Priorität: 30.11.85 DE 3542485
14.08.86 DE 3627667

(43) Veröffentlichungstag der Anmeldung:
10.06.87 Patentblatt 87/24

(84) Benannte Vertragsstaaten:
CH DE FR GB IT LI

(71) Anmelder: BAYER AG
Konzernverwaltung RP Patentabteilung
D-5090 Leverkusen 1 Bayerwerk(DE)

(72) Erfinder: Hassel, Tillmann, Dr.
Morgengraben 14
D-5000 Köln 80(DE)

(72) Erfinder: Hocker, Jürgen, Dr.
Eichenweg 6
D-5060 Bergisch Gladbach 2(DE)

(72) Erfinder: Müller, Hans-Klaus, Dr.
August-Kierspel-Strasse 149
D-5060 Bergisch Gladbach 2(DE)

(72) Erfinder: Fitzky, Hans Georg, Dr.
Adolf-Kolping-Strasse 7
D-5068 Odenthal(DE)

(54) **Radikalanionensalze von Derivaten der 1.4.5.8-Naphthalintetracarbonsäure, Verfahren zu ihrer Herstellung und ihre Verwendung.**

(57) Die Erfindung betrifft kristalline, stabile Radikalanionensalze von Derivaten der 1, 4, 5, 8-Naphthalintetracarbonsäure der Formel

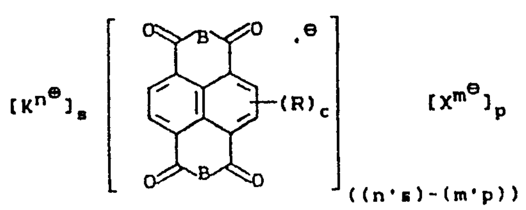

(I)

in der
K, n, s, X, m, p, B, R und c die in der Beschreibung angegebene Bedeutung haben,
Verfahren zu ihrer Herstellung und ihre Verwendung als elektrisch leitende Verbindungen.

EP 0 224 779 A1

BAYER AKTIENGESELLSCHAFT 5090 Leverkusen, Bayerwerk
Konzernverwaltung RP
Patentabteilung B/by-c

Radikalanionensalze von Derivaten der 1.4.5.8-Naphthalintetracarbonsäure, Verfahren zu ihrer Herstellung und ihre Verwendung

Die Erfindung betrifft kristalline, stabile Radikalanionensalze von Derivaten der 1.4.5.8-Naphthalintetracarbonsäure, Verfahren zu ihrer Herstellung und ihre Verwendung als elektrisch leitende Verbindungen.

Aus der DE-OS 29 51 349 sind monomere und aus der DE-OS 30 00 168 (= US-PS 4,368,319) polymere 1.4.5.8-Naphthalintetracarbonsäurebisimide bekannt. Zwar werden beide Naphthalintetracarbonsäure-bisimid-Typen als elektrische Halbleiter und, da ihre elektrische Leitfähigkeit bei Belichtung zunimmt, als Photoleiter beschrieben. Wie jedoch aus Beispiel 4 der DE-OS 30 00 168 hervorgeht und wie die Bestimmung der Gleichstromleitfähigkeiten der bevorzugten und in den Beispielen der DE-OS 29 51 349 beschriebenen Naphthalintetracarbonsäurebisimide ergeben hat, ist die Gleichstromleitfähigkeit sowohl der monomeren als auch der polymeren 1.4.5.8-Naphthalintetracarbonsäurebisimide nur gering.
In der DE-OS 30 00 168 wird in Beispiel 4, dem einzigen Beispiel, in dem sich überhaupt eine Angabe zur Gleichstromleitfähigkeit der polymeren Naphthalintetracarbonsäurebisimide findet, ein Wert von 4,2 x $10^{-8}$ S/cm angegeben. Die Bestimmung der Gleichstromleitfähigkeiten der in den Beispielen beschriebenen monomeren Naphthalintetracarbonsäurebisimide ergab Werte von $10^{-14}$ S/cm, $10^{-11}$S/cm und 9 x $10^{-7}$ S/cm.

Aus J. Am. Chem. Soc. 89, 5925 (1967) ist die Erzeugung von Radikalanionen aus 1.4.5.8-Naphthalintetracarbonsäuredianhydrid und entsprechenden Bis-imiden durch elektrochemische Reduktion bekannt. Die Radikalanionen wurden jedoch nicht in Form von Salzen isoliert, sondern nur in Lösung hergestellt und ESR-spektroskopisch charakterisiert.

Es wurde nun gefunden, daß man Radikalanionen von Derivaten der 1.4.5.8-Naphthalintetracarbonsäure nicht nur in Lösung herstellen, sondern sogar in Form stabiler kristalliner Salze isolieren kann und daß diese stabilen kristallinen Radikalanionensalze eine um mehrere Größenordnungen höhere Gleichstromleitfähigkeit aufweisen als die in den DE-OS 29 51 349 und 30 00 168 beschriebenen 1,4,5,8-Naphthalintetracarbonsäurebisimide. Die spezifischen Leitfähigkeiten dieser Radikalanionensalze liegen zwischen $10^{-5}$ und $10^{-1}$ S/cm. Die kristallinen Radikalanionensalze der 1.4.5.8-Naphthalintetracarbonsäure-Derivate stellen wegen ihrer guten elektrischen Leitfähigkeit und ihrer, verglichen mit den Radikalanionensalzen des 7.7.8.8-Tetracyanochinodimethans (TCNQ) - leichteren Zugänglichkeit eine neue interessante Alternative zu diesen bislang als elektrisch leitende organische Verbindungen verwendeten TCNQ-Komplexen dar.

Die Erfindung betrifft daher kristalline, stabile Radikalanionensalze der Formel

$[K^{n\oplus}]_s$ 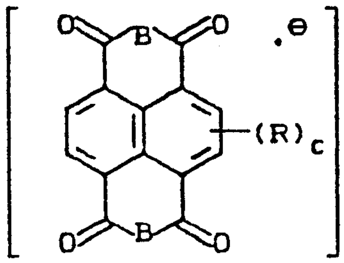 $_{((n \cdot s)-(m \cdot p))}$ $[X^{m\ominus}]_p$ (I)

in der

K für ein n-wertiges Kation steht,

n eine ganze Zahl >0, vorzugsweise eine ganze Zahl von 1 bis 5, besonders bevorzugt 1, 2 oder 3 ist,

s eine ganze Zahl von 1 bis 5 ist,

X für ein m-wertiges Anion steht;

m eine ganze Zahl >0, vorzugsweise 1 oder 2 ist,

p Null, 1 oder eine ganze Zahl >1 ist, mit der Maßgabe, daß (m x p) höchstens den Wert ((n·s)-1) annehmen darf;

B für Sauerstoff oder durch Y substituierten Stickstoff steht, wobei Y ein organischer Rest ist;

R einen Substituenten bedeutet und

c Null oder eine ganze Zahl von 1 bis 4 ist.

Im Falle, daß p eine Zahl >1 ist, kann X auch für verschiedene Anionen stehen.

Diese kristallinen stabilen Radikalanionensalze der Formel (I) können zusätzlich ungeladene Moleküle der ihnen zugrundeliegenden 1.4.5.8-Naphthalintetracarbonsäure-Derivate und/oder Lösungsmittelmoleküle eingeschlossen enthalten. Solche ungeladenen Naphthalintetracarbonsäure-Derivat-Moleküle und/oder Lösungsmittelmoleküle enthaltenden Radikalanionensalze lassen sich durch die Formel

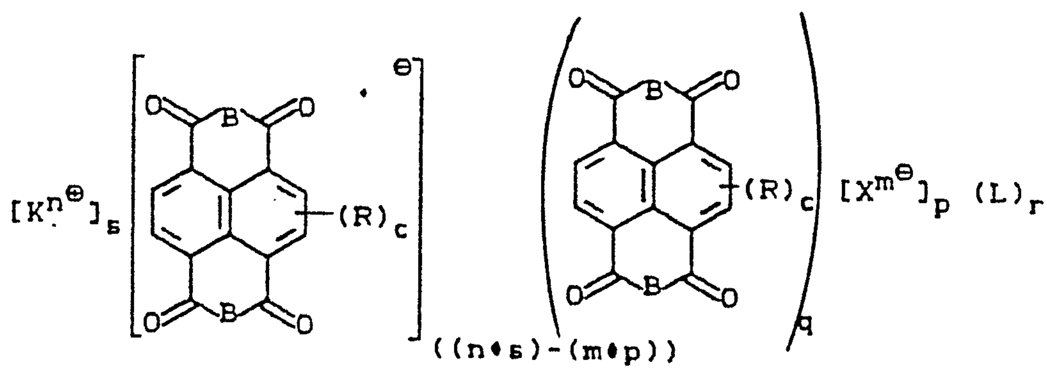

(II)

beschreiben, in der

K, n, s, m, p, X, B, R und c die unter Formel (I) angegebene Bedeutung haben und

q für Null oder eine ganze Zahl von 1 bis 4 steht

L ein Lösungsmittelmolekül bedeutet und

r für Null oder eine ganze Zahl von 1 bis 5 steht.

Als Kationen K kommen folgende Typen von Kationen in Betracht:

1. ein- und zweiwertige anorganische Kationen wie das Natrium-, Kalium-, Rubidium-, Cesium-, Calcium-, Strontium- oder Bariumion.

2. ein- und mehrwertige Oniumionen, wie sie z.B. durch erschöpfende Alkylierung von

- tertiären Phosphinen wie Trimethylphosphin, Tributylphosphin und Triphenylphosphin;
- tertiären Arsinen wie Tributylarsin, Trimethylarsin und Triphenylarsin;
- Thioethern wie Dimethylsulfid, Dipropylsulfid und Diethylsulfid sowie
- tertiären aliphatischen, alicyclischen, aromatischen und heterocyclischen Mono- und Polyaminen

erhalten werden.

Als Alkylierungsmittel für die erschöpfende Alkylierung seien beispielsweise genannt: Iodmethan, Chlormethan, Bromethan, 1.2-Dibromethan, 1,3-Dibrompropan, Dimethylsulfat und Diethylsulfat. Bevorzugtes Alkylierungsmittel ist Dimethylsulfat. Bevorzugte Kationen sind die durch erschöpfende Alkylierung von tertiären Mono- und Polyaminen erhältlichen Mono- und Polyammonium-Kationen.

Als tertiäre Mono- und Polyamine können verwendet werden

- aliphatische Amine wie
  N.N.N'.N'-Tetramethyl-1.2-diaminoethan, N.N.N'.N'-Tetramethyl-1.3-diaminopropan, N.N.N'.N'-Tetramethyl-1.4-diaminobutan, N.N.N'.N'-Tetramethyl-1.6-diaminohexan, N.N.N'.N".N"-Pentamethyl-1.5-diamino-3-aza-pentan und N.N.N'.N".N"-Pentamethyl-1.7-diamino-4-aza-heptan

- alicyclische Amine wie
  N.N'-Dimethylpiperazin und 1.4-Diaza-[2.2.2]-bicyclooctan

- aromatische Amine wie
  Dimethylanilin, N.N.N'.N'-Tetramethyl-4.4'-diaminodiphenyl

- heterocyclische Amine wie
  Pyridin, Lutidin, Collidin, Chinolin und Isochinolin.

Bevorzugte tertiäre Amine sind
N.N.N'.N'-Tetramethyldiaminoethan, N.N.N'.N'-Tetramethyl-1.3-diaminopropan, N.N.N'.N'-Tetramethyl-1.4-diaminobutan, N.N.N'.N".N"-Pentamethyl-1.7-diamino-4-aza-heptan, 1.4-Diaza-[2.2.2]-bicyclo-octan, N.N'-Dimethylpiperazin, Pyridin und Chinolin.

Als dritter Kationen-Typ seien Carbenium-Ionen genannt, wie sie bei der Oxidation von elektronenreichen Olefinen wie

Tetraaminoethylenen; z.B. Bis-1,3-diphenylimidazolidinyliden-(2), Tetrathioethylenen; z.B. Tetrathiafulvalen oder Diamino-dithio(dioxo)-ethylenen; z.B. Bis-3-methylthiazolinyliden-(2)

erhalten werden.

Das Anion X kann einwertig, wie das Chlorid-, Bromid-, Monoethylsulfat-, Monomethylsulfat-, Perchlorat-, Tetrafluoroborat- oder Hexafluorophosphat-Ion aber auch zweiwertig wie das Sulfat-, und dreiwertig wie das Phosphat-Ion sein. Bevorzugt sind einwertige Anionen wie das Monomethylsulfat- und Tetrafluoroborat-Anion.

Der organische Rest Y in B kann ein gegebenenfalls substituierter Alkyl-, Cycloalkyl-, Aralkyl- oder Arylrest sein; bevorzugt sind $C_1$-$C_6$-Alkylreste wie der Methyl-, Ethyl-, i-Propyl-, n-Butyl- und sec.-Butyl-Rest, ferner gegebenenfalls substituierte Arylreste wie der Phenyl-, Nitrophenyl-, Cyanophenyl-, Halogenphenyl-, $C_1$-$C_4$-Alkoxyphenyl- und $C_1$-$C_4$-Alkylphenyl-Rest.

Als Substituent R seien beispielsweise genannt Halogenatome wie Fluor, Chlor oder Brom; ferner Nitro-, Hydroxy-, Cyano-, $C_1$-$C_4$-Alkyl-, Cyclohexyl-, Phenyl- oder Carbonsäureestergruppen.

Besonders bevorzugt sind solche Verbindungen der Formel (I), wenn in dieser B für Sauerstoff oder ein durch Y substituiertes Stickstoffatom steht, wobei Y ein gegebenenfalls substituierter $C_1$-$C_6$-Alkylrest wie der Methyl-, Ethyl-, n-Propyl-, sec.-Butyl- oder n-Hexyl-Rest oder einen 2-(Trimethylammonium-)ethyl-, 3-(Trimethylammonium-)propyl-Rest oder ein gegebenenfalls ein- oder mehrfach substituierter einkerniger Arylrest, wie der Phenyl-, Tolyl-, Anisyl-, Dimethylaminophenyl-, Chlorphenyl-, Dichlorphenyl-, Nitrophenyl-, Cyanophenyl- oder $C_1$-$C_4$-Alkoxycarbonylphenyl-Rest, ist.

Die Erfindung betrifft ferner Verfahren zur Herstellung der kristallinen stabilen Radikalanionensalze der Formel (I) bzw. (II). Die Verfahren gehen von 1.4.5.8-Naphthalintetracarbonsäure-Derivaten der Formel

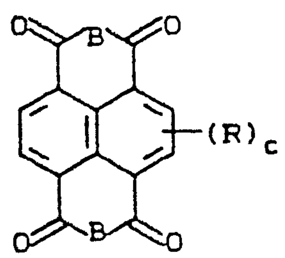

(III)

aus, in der

B, R und c die unter Formel (I) angegebene Bedeutung haben.

Diese Derivate werden entweder chemisch oder - vorzugsweise - elektrochemisch in einem inerten organischen Lösungsmittel reduziert. Die bei den Reduktionsverfahren anfallenden Radikalanionensalze der Formel (I) (bzw. II) scheiden sich in Form fester kristallisierter Verbindungen ab. Diese kristallinen Verbindungen werden mechanisch von der flüssigen Phase abgetrennt, mit dem verwendeten Lösungsmittel gewaschen und getrocknet.

Bei der elektrochemischen Reduktion der 1.4.5.8-Naphthalintetracarbonsäure-Derivate werden diese in einem inerten Lösungsmittel in Gegenwart eines Leitsalzes der Formel

$$[K^{n\ominus}]_s \quad [X^{m\ominus}]_{p'}$$

in der

K, n, s, X und m die unter Formel (I) angegebene Bedeutung haben und

p' eine ganze Zahl >1 ist, mit der Maßgabe, daß (n·s) = (m·p) ist,

reduziert. Diese elektrochemische Reduktion sei an folgendem konkreten Beispiel erläutert:

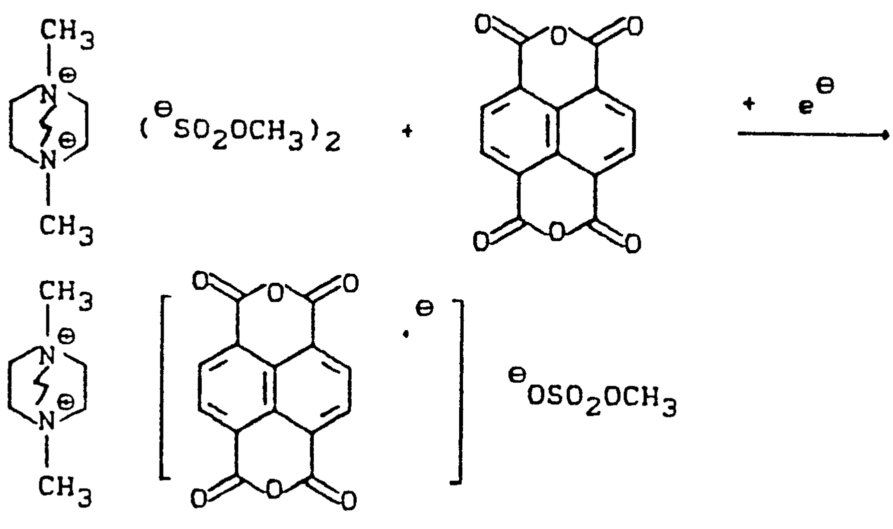

Als inerte Lösungsmittel können verwendet werden:

- Nitrile wie Acetonitril, Propionitril und Butyronitril
- Amide wie Dimethylformamid und Dimethylacetamid
- Harnstoffe wie Tetramethylharnstoff
- Carbonate wie 1.3-Dioxa-cyclohexanon-(2) und 1,3-Dioxa-4-methyl-cyclopentanon-(2)
- Lactone und Lactame wie Butyrolacton und N-Methylpyrrolidon
- Sulfoxide und Sulfone wie Dimethylsulfoxid und Dimethylsulfon und Tetramethylensulfon.

Bevorzugt sind Nitrile, Amide und Carbonate wie Dimethylformamid, Acetonitril und 1.3-Dioxa-cyclohexanon-(2) und 1,3-Dioxa-4-methyl-cyclopentanon-(2).

Als Leitsalze werden die Chloride, Bromide, Monomethyl-sulfate, Perchlorate, Tetrafluoroborate und Hexafluoro-phosphate der vorstehend genannten anorganischen Kationen, oder der durch erschöpfende Alkylierung der Arsine, Phosphine, Thioether und tertiären Amine erhaltenen Oniumsalze verwendet.

Diese Oniumsalze können unmittelbar in der Form verwendet werden, in der sie bei der erschöpfenden Alkylierung anfallen. Man kann in ihnen aber auch zunächst das Anion gegen ein anderes Anion austauschen, z.B. im Tetrabutylammoniumchlorid, das Chloridion gegen das Tetrafluoroboration.

Bevorzugte Leitsalze sind die Methosulfate und Tetrafluoroborate der durch erschöpfende Methylierung von N.N.N'.N'-Tetramethyl-1.2-diaminoethan, N.N.N'.N'-Tetramethyl-1.3-diaminopropan, N.N.N'.N'-Tetramethyl-1.4-diaminobutan, N.N.N'.N".N"-Pentamethyl-1.7-diamino-4-azaheptan, N.N'-Dimethylpiperazin, 1.4-Diaza-[2.2.2]-bicyclooctan, Pyridin und Chinolin erhaltenen Mono- und Polykationen.

Die elektrochemische Reduktion wird bei Temperaturen von -40°C bis zum Siedepunkt des verwendeten Lösungsmittels vorgenommen. Bevorzugt sind Temperaturen von 0 bis 100°C, insbesondere Temperaturen von 20°C bis 85°C.

Die Derivate der 1.4.5.8-Naphthalintetracarbonsäure können in einer Konzentration von 0,005 Mol/l bis zur Sättigungskonzentration für die verwendete Temperatur im jeweiligen Lösungsmittel eingesetzt werden. Bevorzugt sind Konzentrationen von 0,005 bis 0,02 Mol/l.

Die jeweiligen Leitsalze können im zwei- bis zwanzigfachen molaren Überschuß, bezogen auf eingesetztes Tetracarbonsäurederivat, verwendet werden. Bevorzugt werden 3 bis 12 Mol Leitsalz je Mol Tetracarbonsäure-Derivat eingesetzt.

Die elektrochemische Reduktion kann potentiostatisch oder galvanostatisch durchgeführt werden. Bevorzugt wird die galvanostatische Arbeitsweise. In einem typischen galvanostatischen Experiment wird in einer 100 ml-Zelle mit 2 in einem Abstand von 1 cm angeordneten 16 $cm^2$-Pt-Elektroden bei einer Stromstärke von 2 mA und einer Zellspannung

zwischen 0,7 V und 3 V elektrolysiert. Gegebenenfalls können Anoden- und Kathodenraum durch eine Membran oder Fritte getrennt sein.

Die erfindungsgemäßen Radikalanionensalze der Formel I scheiden sich während der Elektrolyse an der Kathode ab und können in an sich bekannter Weise durch mechanisches Abtrennen, Waschen mit einem der oben erwähnten inerten Lösemittel und Trocknen analysenrein gewonnen werden.

Bei der chemischen Reduktion werden die Naphthalintetracarbonsäurederivate mit einem chemischen Reduktionsmittel umgesetzt. Geeignete chemische Reduktionsmittel sind z.B. anorganische Verbindungen wie Alkalimetalle; bevorzugte Reduktionsmittel sind organische elektronenreiche Verbindungen wie z.B. Bis-1.3-diphenylimidazolidinyliden-(2) oder Bis-3-methylbenzthiazolinyliden-(2). Die chemische Umsetzung wird bevorzugt in einem polaren organischen Lösungsmittel wie Acetonitril, Dimethylformamid, Sulfolan, Propylencarbonat oder N-Methylpyrrolidon durchgeführt.

Die chemische Reduktion wird bei Temperaturen von 20°C bis zum Siedepunkt des verwendeten Lösungsmittels ausgeführt. Durch Anwendung von Druck können jedoch auch höhere Reaktionstemperaturen angewandt werden. Nach dem Abkühlen fällt das halbleitende Radikalionensalz in kristalliner Form aus.

Die erfindungsgemäßen Radikalionensalze der Formeln I bzw. II sind wertvolle Halbleiter. Sie finden Verwendung zur Antistatikausrüstung von Kunststoffen und auf dem Elektroniksektor.

Beispiel 1

In einer heizbaren 100 ml Elektrolysezelle mit zwei 16 $cm^2$-Pt-Elektroden im Abstand von 1 cm wird eine Lösung von 350 mg (1,306 mmol) 1.4.5.8-Naphthalintetracarbonsäuredianhydrid und 1,59 g (5 mmol) N.N.N'.N'-Tetramethylpiperazinium-bis-tetrafluoroborat in 100 ml Dimethylformamid bei 80° C vorgelegt. Man elektrolysiert 26 Stunden bei 1,5 mA, wobei sich eine Zellenspannung von 1,4 V einstellt. An der Kathode kristallisiert das Radikalanionensalz in Form blauschwarzer, metallisch glänzender Nadeln aus. Es wird abgesaugt, mit Dimethylformamid, dann mit Acetonitril gewaschen und getrocknet.

Ausbeute: 280 mg (= 0,4117 mmol = 62 % der Theorie) Radikalanionensalz der Formel

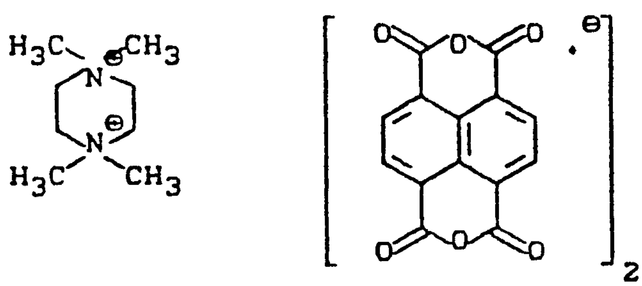

Beispiel 2

In einer heizbaren 100 ml Elektrolysezelle mit zwei 16 $cm^2$-Pt-Elektroden im Abstand von 1 cm wird eine Lösung von 268 mg (1 mmol) 1.4.5.8-Naphthalintetracarbonsäuredianhydrid und 3,32 g (9 mmol) N,N'-Dimethyl-diaza-[2.2.2]-bicyclooctan-bis-methosulfat in 100 ml Dimethylformamid bei 80° C vorgelegt. Man elektrolysiert 26 Stunden bei 1,5 mA, wobei sich eine Zellenspannung von 1,5 V einstellt. An der Kathode kristallisiert das Radikalanionensalz in Form blauschwarzer, metallisch glänzender Nadeln aus. Es wird abgesaugt, mit Dimethylformamid, dann mit Acetonitril gewaschen und getrocknet.

Ausbeute: 277 mg (= 0,35 mmol = 35 % der Theorie) Radikalanionensalz der Formel

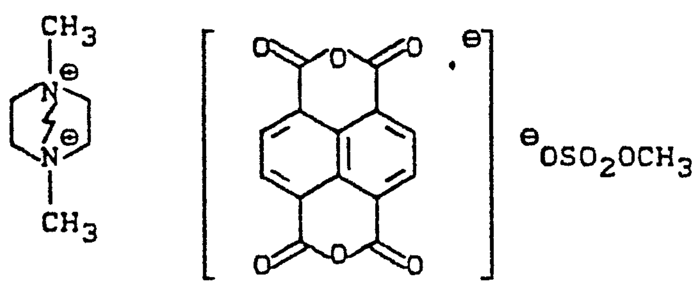

Die Leitfähigkeit des Salzes beträgt $4 \cdot 10^{-3}$ S/cm (Pulverpreßling, Zweielektrodenmethode).

Beispiel 3

In einer heizbaren 100 ml Elektrolysezelle mit zwei 16 $cm^2$-Pt-Elektroden im Abstand von 1 cm wird eine Lösung von 0,545 mg (1,3 mmol) N,N'-Diphenyl-naphthalintetracarbonsäurebisimid und 2,55 g (10 mmol) N-Methylchinoliniummethosulfat in 100 ml Dimethylformamid bei 80° C vorgelegt. Man elektrolysiert 13 Stunden bei 1,5 mA, wobei sich eine Zellenspannung von 1,35 V einstellt. An der Kathode kristallisiert das Radikalanionensalz in Form blauschwarzer, metallisch glänzender Nadeln aus. Es wird abgesaugt, mit Dimethylformamid, dann mit Acetonitril gewaschen und getrocknet.

Ausbeute: 140 mg (= 0,13 mmol = 20 % der Theorie) Radikalanionensalz der Formel

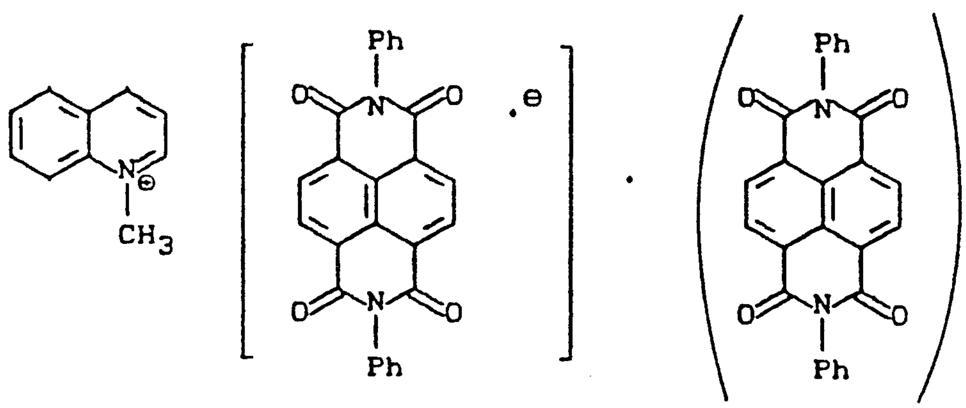
.DMF

Die Leitfähigkeit des Salzes beträgt 6,9 · $10^{-4}$ S/cm (Pulverpreßling, Zweielektrodenmethode).

Beispiel 4

In einer heizbaren 100 ml Elektrolysezelle mit zwei 16 $cm^2$-Pt-Elektroden im Abstand von 1 cm wird eine Lösung von 0,545 mg (1,3 mmol) N,N'-Diphenyl-naphthalintetracarbonsäurebisimid und 1,6 g (5 mmol) N,N'-Dimethyl-diaza-[2.2.2]-bicyclooctan-bis-tetrafluoroborat in 100 ml Dimethylformamid bei 80° C vorgelegt. Man elektrolysiert 8 Stunden bei 1,5 mA; wobei sich eine Zellenspannung von 1,4 V einstellt. An der Kathode kristallisiert das Radikalanionensalz in Form blauschwarzer, metallisch glänzender Nadeln aus. Es wird abgesaugt, mit Dimethylformamid, dann mit Acetonitril gewaschen und getrocknet.

Ausbeute: 176 mg (= 0,08 mmol = 18 % der Theorie) Radikalanionensalz der Formel

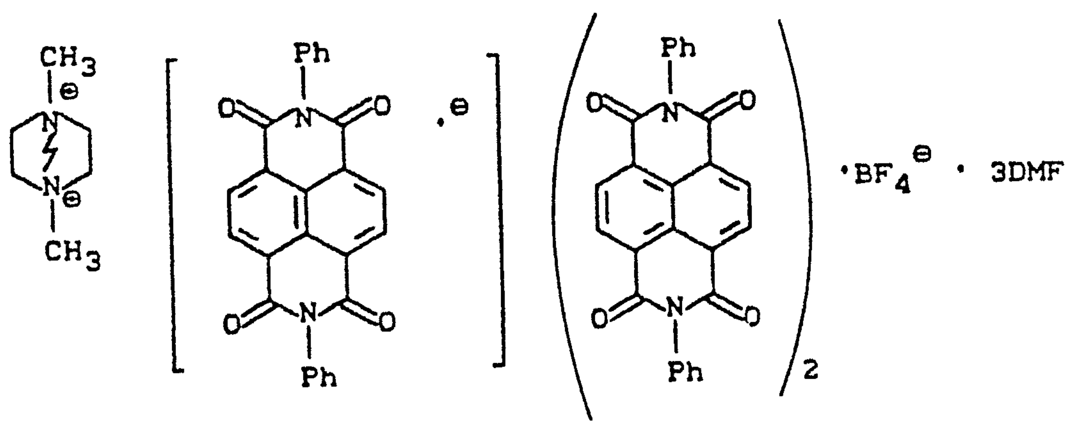

Die Leitfähigkeit des Salzes beträgt 4 · $10^{-4}$ S/cm (Pulverpreßling, Zweielektrodenmethode).

Beispiel 5

In einer heizbaren 100 ml Elektrolysezelle mit zwei 16 $cm^2$-Pt-Elektroden im Abstand von 1 cm wird eine Lösung von 0,545 mg (1,3 mmol) N,N'-Diphenyl-naphthalintetracarbonsäurebisimid und 1,6 g (0,5 mmol) N,N,N,N',N',N'-Hexamethylethylenbisammonium-bis-tetrafluoroborat in 100 ml Dimethylformamid bei $80^{\circ}C$ vorgelegt. Man elektrolysiert 14 Stunden bei 1,5 mA; wobei sich eine Zellenspannung von 1,4 V einstellt. An der Kathode kristallisiert das Radikalanionensalz in Form blauschwarzer, metallisch glänzender Nadeln aus. Es wird abgesaugt, mit Dimethylformamid, dann mit Acetonitril gewaschen und getrocknet.

Ausbeute: 96 mg (= 0,05 mmol = 15 % der Theorie) Radikalanionensalz der Formel

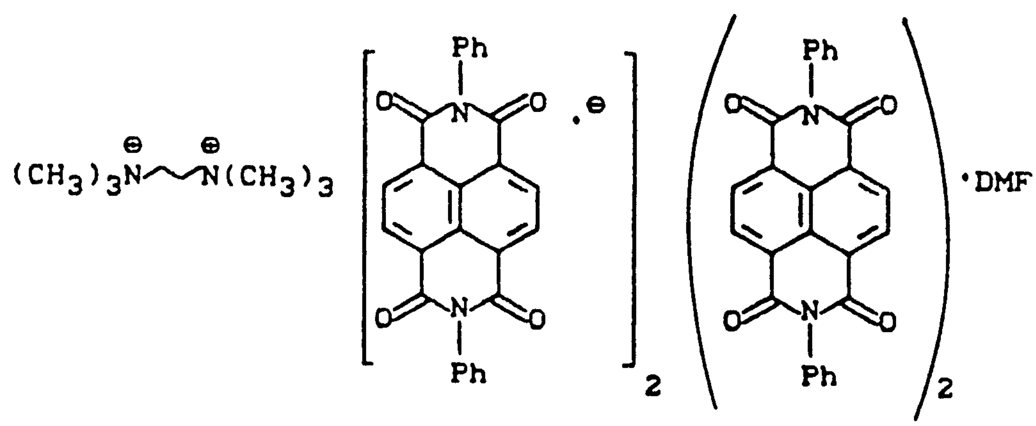

Die Leitfähigkeit des Salzes beträgt $7,5 \cdot 10^{-2}$ S/cm (Pulverpreßling, Zweielektrodenmethode).

Beispiele 6 bis 23

Es wurde wie in Beispiel 1 beschrieben verfahren, nur wurden anstelle des verwendeten Leitsalzes und Lösungsmittels die in der nachstehenden Tabelle angegebenen Leitsalze und Lösungsmittel verwendet.

In der nachstehenden Tabelle sind die in den Beispielen 6 bis 23 verwendeten Leitsalze, Lösungsmittel und die erhaltenen Radikalanionensalze der 1.4.5.8-Naphthalintetracarbonsäure-Derivate zusammengestellt.

Die erhaltenen Radikalanionensalze werden durch die Formel (II) charakterisiert; in der Tabelle sind die Bedeutungen von B, R und X und die Werte für n, m, p, q, r und c aufgeführt, die diese in Formel (II) für die einzelnen Radikalanionensalze annehmen.

Die in der Tabelle für die Kennzeichnung der Lösungsmittel verwendeten Abkürzungen haben folgende Bedeutung:

| | |
|---|---|
| DMF | Dimethylformamid |
| PPC | Propylen-1,2-carbonat |
| AC | Acetonitril |
| NMP | N-Methylpyrrolidon |

**Tabelle**

| Bsp. Nr. | Leitsalz Kation | Anion | LM | B | R | c | n | s | ZRA* | q | m | p | X | r |
|---|---|---|---|---|---|---|---|---|---|---|---|---|---|---|
| 6 | 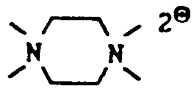 | $(OSO_2OCH_3^{\ominus})_2$ | DMF | Sauerstoff | - | 0 | 2 | 1 | 2 | 0 | - | 0 | - | 0 |
| 7 | 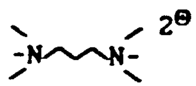 | " | DMF | " | - | 0 | 2 | 1 | 2 | 0 | - | 0 | - | 0 |
| 8 | 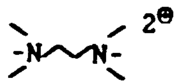 | " | DMF | " | - | 0 | 2 | 1 | 2 | 0 | - | 0 | - | 0 |
| 9 | 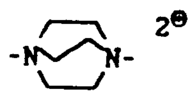 | " | PPC | " | - | 0 | 2 | 1 | 1 | 0 | 1 | 1 | $OSO_2OCH_3^{\ominus}$ | 0 |
| 10 | " | $(BF_4^{\ominus})_2$ | PPC | " | - | 0 | 2 | 1 | 2 | 0 | - | 0 | - | 0 |
| 11 | " | " | PPC | " | - | 0 | 2 | 1 | 2 | 0 | - | 0 | - | 0 |

*) ZRA = (n·s)-(m·p) (= Zahl der Radikalanionen)

Tabelle (Fortsetzung)

| Bsp. Nr. | Leitsalz Kation | Anion | LM | B | R | c | n | s | ZRA | q | m | p | X | r |
|---|---|---|---|---|---|---|---|---|---|---|---|---|---|---|
| 12 | 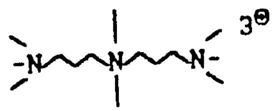 | $(BF_4^{\ominus})_3$ | DMF | Sauerstoff | - | 0 | 3 | 1 | 3 | 0 | - | 0 | - | 0 |
| 13 | 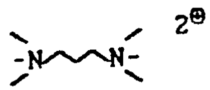 | $(BF_4^{\ominus})_2$ | DMF | " | - | 0 | 2 | 1 | 2 | 0 | - | 0 | - | 0 |
| 14 | 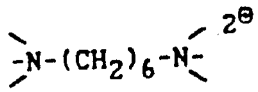 | " | PPC | " | - | 0 | 2 | 1 | 2 | 0 | - | 0 | - | 0 |
| 15 | 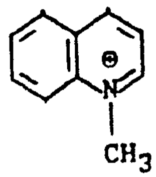 | $OSO_2OCH_3^{\ominus}$ | PPC | " | - | 0 | 1 | 1 | 1 | 0 | - | 0 | - | 0 |
| 15 | " | " | AC | " | - | 0 | 1 | 1 | 1 | 0 | - | 0 | - | 0 |
| 16 | 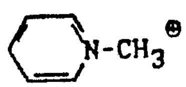 | " | AC | " | - | 0 | 1 | 1 | 1 | 0 | - | 0 | - | 0 |
| 17 | " | " | PPC | " | - | 0 | 1 | 1 | 1 | 0 | - | 0 | - | 0 |
| 18 | 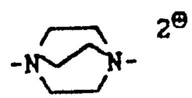 | $(BF_4^{\ominus})_2$ | DMF | $N-CH_3$ | - | 0 | 2 | 1 | 2 | 0 | - | 0 | - | 0 |

Tabelle (Fortsetzung)

| Bsp. Nr. | Leitsalz Kation | Anion | LM | B | R | c | n | s | ZRA | q | m | p | X | r |
|---|---|---|---|---|---|---|---|---|---|---|---|---|---|---|
| 20 | 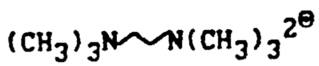 | $(BF_4^{\ominus})_2$ | DMF | 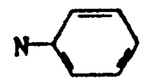 | - | 0 | 2 | 1 | 2 | 2 | - | 0 | - | 0 |
| 21 | 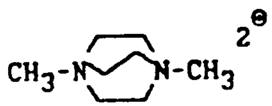 | $(OSO_2OCH_3^{\ominus})_2$ | PPC | Sauerstoff | Cl | 4 | 2 | 1 | 2 | 0 | 1 | 1 | $OSO_2OCH_3^{\ominus}$ | 2 |
| 22 | " | $(BF_4^{\ominus})_2$ | NMP | 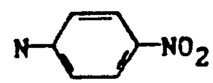 | - | 0 | 2 | 3 | 2 | 0 | 1 | 4 | $BF_4^{\ominus}$ | 0 |
| 23 | " | $(BF_4^{\ominus})_2$ | PPC | 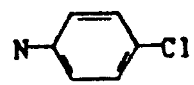 | - | 0 | 2 | 3 | 2 | 0 | 1 | 4 | $BF_4^{\ominus}$ | 2 |

Beispiel 24

In einer heizbaren 100 ml-Elektrolysezelle, die mit zwei in einem Abstand von 1,5 cm befindlichen 16 $cm^2$-Platin-Elektroden ausgerüstet ist und deren Anoden- und Kathodenraum durch eine Fritte voneinander getrennt sind, wird eine Lösung von 1,66 g Hexamethylethylendiaminbistetrafluoroborat in 100 ml Dimethylformamid vorgelegt. In den Anodenraum werden 0,894 g N,N'-Bis-(3-trimethylammonium-propyl)-naphthalintetracarbonsäurebisimid-bismethosulfat eingetragen. Die Lösung wird auf 80° C erwärmt und dabei gründlich mit Stickstoff gespült. Man elektrolysiert 22 Stunden bei 1,5 mA; dabei stellt sich eine Zellspannung von 2,0 V ein. An der Kathode kristallisiert das Radikalanionensalz in Form braunschwarzer Nadelbüschel aus, die schließlich zu einem Elektrodenüberzug zusammenwachsen. Nach beendeter Elektrolyse wird der Elektrodenüberzug mehrfach mit Acetonitril gewaschen und getrocknet. Er zeigt eine Leitfähigkeit von $10^{-5}$ S/cm.

Ausbeute: 0,248 g

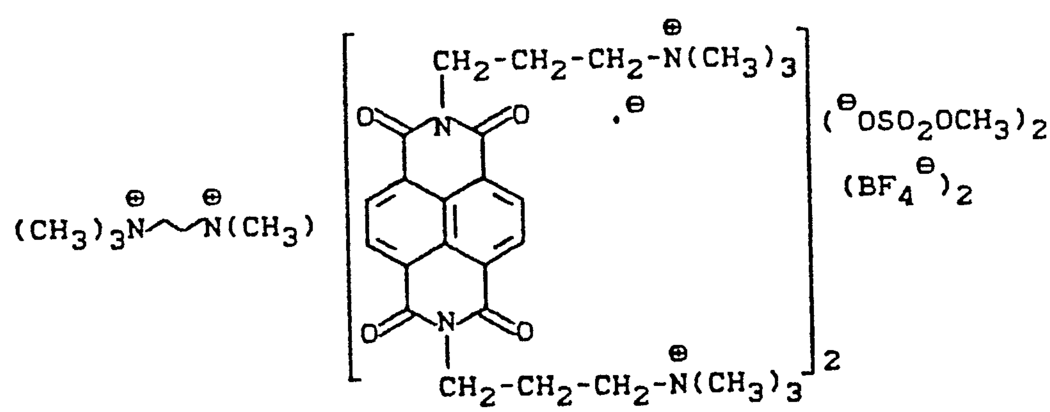

Beispiel 25

2,22 g Bis-1.3-diphenylimidazolidinyliden-(2) werden unter Stickstoff in 50 ml siedendem Dimethylformamid gelöst und mit einer Lösung von 4,02 g Naphthalintetracarbonsäuredianhydrid in 150 ml siedendem Dimethylformamid unter Stickstoff vereinigt.

Beim Abkühlen fallen 2,25 g violettschwarze, metallisch glänzende Kristalle der Zusammensetzung aus:
C = 69,3 %; H = 3,3 %; N = 4,4 %; O = 23,0 %.

Die Substanz zeigt eine spezifische elektrische Leitfähigkeit von 6 x $10^{-3}$ S/cm.

Beispiel 26

Analog Beispiel 25 wurden 2,8 g Bis-[1,3-(p-dichlorphenyl)imidazolidinyliden-(2)] mit 4,02 g Naphthalintetracarbonsäuredianhydrid umgesetzt.

Man erhält 2,4 g dunkelbraune Kristalle mit einer spezifischen Leitfähigkeit von 1,2 x $10^{-4}$ S/cm.

Patentansprüche

1. Kristalline, stabile Radikalanionensalze der Formel

$[K^{n\oplus}]_s$ 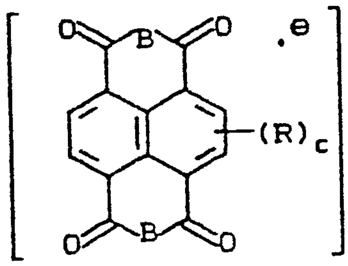 $((n \cdot s)-(m \cdot p))$ $[X^{m\ominus}]_p$ (I)

in der

K für ein n-wertiges Kation steht,

n eine ganze Zahl >0 ist,

s eine ganze Zahl von 1 bis 5 ist,

X für ein m-wertiges Anion steht;

m eine ganze Zahl >0 ist,

p Null, 1 oder eine ganze Zahl >1 ist, mit der Maßgabe, daß $(m \cdot p)$ höchstens den Wert $((n \cdot s)-1)$ annehmen darf;

B für Sauerstoff oder durch Y substituierten Stickstoff steht, wobei Y ein organischer Rest ist;

R einen Substituenten bedeutet und

c Null oder eine ganze Zahl von 1 bis 4 ist.

2. Kristalline, stabile Radikalanionensalze gemäß Anspruch 1, dadurch gekennzeichnet, daß in ihnen

n eine ganze Zahl von 1 bis 5 ist,

m 1 oder 2 ist,

Y ein gegebenenfalls substituierter Alkyl-, Cycloalkyl-, Aralkyl- oder Arylrest ist und

R für ein Halogenatom oder eine Nitro-, Hydroxy-, Cyano-, $C_1$-$C_4$-Alkyl-, Cyclohexyl-, Phenyl- oder Carbonsäureester-Gruppe steht.

3. Kristalline, stabile Radikalanionensalze gemäß Anspruch 2, dadurch gekennzeichnet, daß in ihnen

n 1, 2 oder 3 ist.

4. Radikalanionensalze gemäß Anspruch 1, 2 oder 3, dadurch gekennzeichnet, daß sie der Formel

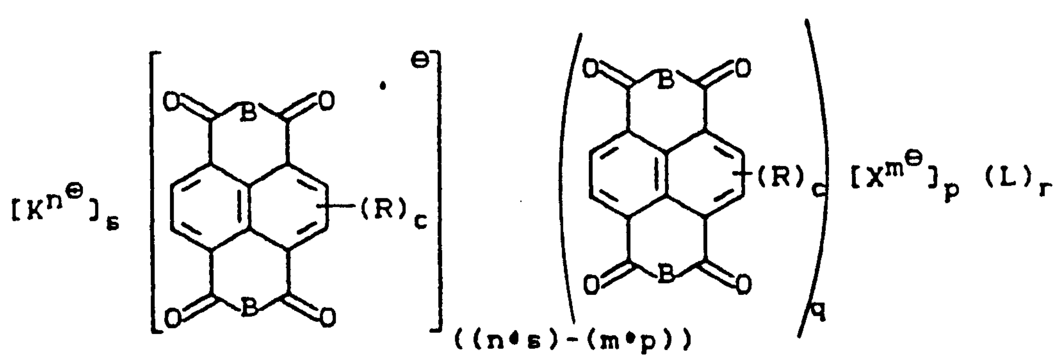

(II)

entsprechen, in der

K, n, s, m, p, X, B, R und c die in Anspruch 1, 2 oder 3 angegebene Bedeutung haben und

q    für Null oder eine ganze Zahl von 1 bis 4 steht

L    ein Lösungsmittelmolekül bedeutet und

r    für Null oder eine ganze Zahl von 1 bis 5 steht.

5. Verfahren zur Herstellung von Radikalanionensalzen der Formel

$[K^{n\ominus}]_s$ 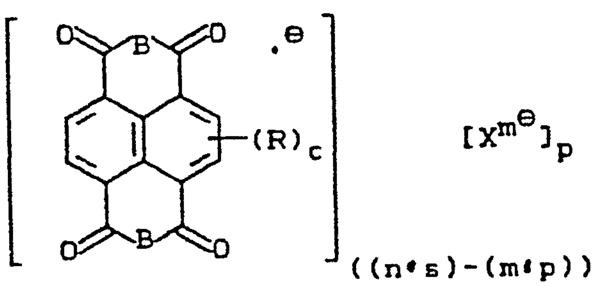 $[X^{m\ominus}]_p$    (I)

in der

K    für ein n-wertiges Kation steht,

n    eine ganze Zahl >0 ist,

s eine ganze Zahl von 1 bis 5 ist,

X für ein m-wertiges Anion steht;

m eine ganze Zahl >0 ist,

p Null, 1 oder eine ganze Zahl >1 ist, mit der Maßgabe, daß (m•p) höchstens den Wert ((n•s)-1) annehmen darf;

B für Sauerstoff oder durch Y substituierten Stickstoff steht, wobei Y ein organischer Rest ist;

R einen Substituenten bedeutet und

c Null oder eine ganze Zahl von 1 bis 4 ist,

dadurch gekennzeichnet, daß man 1.4.5.8-Naphthalintetracarbonsäurederivate der Formel

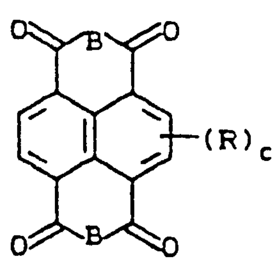

(III)

in der

B, R und c die vorstehend unter Formel (I) angegebene Bedeutung haben,

0224779

in einem inerten organischen Lösungsmittel chemisch oder elektrochemisch reduziert.

6. Verfahren gemäß Anspruch 5, dadurch gekennzeichnet, daß

n eine ganze Zahl von 1 bis 5 ist,

m 1 oder 2 ist,

Y ein gegebenenfalls substituierter Alkyl-, Cycloalkyl-, Aralkyl- oder Arylrest ist und

R für ein Halogenatom oder eine Nitro-, Hydroxy-, Cyano-, $C_1$-$C_4$-Alkyl-, Cyclohexyl-, Phenyl- oder Carbonsäureester-Gruppe steht.

7. Verfahren gemäß Anspruch 5, dadurch gekennzeichnet, daß man die 1.4.5.8-Naphthalincarbonsäure-Derivate in einem inerten Lösemittel in Gegenwart eines Leitsalzes der Formel

$$[K^{n\ominus}]_s\ [X^{m\ominus}]_p, \qquad (IV)$$

in der

K für ein n-wertiges Kation steht,

n eine ganze Zahl >0 ist,

s eine ganze Zahl von 1 bis 5 ist,

X für ein m-wertiges Anion steht,

m eine ganze Zahl >0 ist und

p' eine ganze Zahl >1 ist mit der Maßgabe, daß $(n \cdot s) = (m \cdot p')$ ist

bei Zimmertemperatur oder erhöhter Temperatur elektrochemisch reduziert.

8. Verwendung der Radikalanionensalze gemäß Anspruch 1, 2, 3 oder 4 zur Antistatikausrüstung von Kunststoffen.

0224779

Europäisches Patentamt

# EUROPÄISCHER RECHERCHENBERICHT

Nummer der Anmeldung

EP 86 11 5879

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
|---|---|---|---|
| D,A | JOURNAL OF THE AMERICAN CHEMICAL SOCIETY, Band 89, Nr. 23, 8. November 1967, pages 5925-5931, Washington DC, US; S.F. NELSEN "Heterocyclic radical anions. II. Naphthalic and 1,4,5,8-Naphthalenetetracarboxylic acid derivatives" * Seite 5927, Figur 4 * | 1 | C 07 D 493/06<br>C 07 D 471/06<br>C 08 K 5/15<br>C 08 K 5/34 //<br>(C 07 D 493/06<br>C 07 D 311:00<br>C 07 D 311:00)<br>(C 07 D 471/06<br>C 07 D 221:00<br>C 07 D 221:00) |
| D,A | DE-A-3 000 168 (BAYER AG) * Ansprüche 1, 3 * | 1 | |
| D,A | DE-A-2 951 349 (BAYER AG) * Anspruch * | 1 | |
| A | CHEMICAL ABSTRACTS, Band 97, Nr. 26, 27. Dezember 1982, Seite 826, Spalte 1, Zusammenfassungsnr. 228129n, Columbus, Ohio, US; S.R. FORREST et al.: "Large conductivity changes in ion-beam-irradiated organic thin films", & APPL. PHYS. LETT. 1982, 41(8), 708-710 | 1 | **RECHERCHIERTE SACHGEBIETE (Int. Cl 4)**<br>C 07 D 471/06<br>C 07 D 493/06<br>C 08 K 5/00 |
| A | US-A-3 507 871 (F. IRVING et al.) * Spalte 2, Zeilen 26-38, Formel * | 1 | |

--- -/-

Der vorliegende Recherchenbericht wurde für alle Patentansprüche erstellt

| Recherchenort | Abschlußdatum der Recherche | Prüfer |
|---|---|---|
| BERLIN | 18-02-1987 | HASS C V F |

KATEGORIE DER GENANNTEN DOKUMENTE
- X : von besonderer Bedeutung allein betrachtet
- Y : von besonderer Bedeutung in Verbindung mit einer anderen Veröffentlichung derselben Kategorie
- A : technologischer Hintergrund
- O : nichtschriftliche Offenbarung
- P : Zwischenliteratur
- T : der Erfindung zugrunde liegende Theorien oder Grundsätze
- E : älteres Patentdokument, das jedoch erst am oder nach dem Anmeldedatum veröffentlicht worden ist
- D : in der Anmeldung angeführtes Dokument
- L : aus andern Gründen angeführtes Dokument
- & : Mitglied der gleichen Patentfamilie, übereinstimmendes Dokument

EPA Form 1503 03 82

Europäisches Patentamt

**0224779**

# EUROPÄISCHER RECHERCHENBERICHT

Nummer der Anmeldung

EP 86 11 5879

Seite 2

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int Cl 4) |
| --- | --- | --- | --- |
| A | EP-A-0 093 967 (BASF AG)<br>* Ansprüche 9, 15 *<br>----- | 8 | |

RECHERCHIERTE SACHGEBIETE (Int. Cl.4)

Der vorliegende Recherchenbericht wurde für alle Patentansprüche erstellt.

| Recherchenort | Abschlußdatum der Recherche | Prüfer |
| --- | --- | --- |
| BERLIN | 18-02-1987 | HASS C V F |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur
T : der Erfindung zugrunde liegende Theorien oder Grundsätze

E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPA Form 1503 03 82